# EUROPEAN PATENT APPLICATION

(11) **EP 4 285 873 A1**
(43) Date of publication of application: **06.12.2023**
(21) Application number: 23175326.0
(22) Date of filing: 25.05.2023
(51) Int. Cl.: A61F 2/95, A61F 2/954, A61F 2/07

(54) **DELIVERY SYSTEM FOR A VASCULAR PROSTHESIS**

(30) Priority: 31.05.2022 DE 102022113756
(71) Applicant: JOTEC GmbH, 72379 Hechingen (DE)
(72) Inventor: Klein, Karsten, 72116 Mössingen (DE)
(74) Representative: Witte, Weller & Partner Patentanwälte mbB

(57) **Abstract**

The present invention relates to a system for delivery of an endoluminal prosthesis, the system comprising a delivery catheter and a prosthesis to be delivered, wherein the system comprises a wire extending through at least a portion of a main lumen of the prosthesis in a preloaded configuration, the wire extending in distal direction to a position in proximity to the distal opening of the prosthesis and the wire extending in proximal direction of the proximal opening of the prosthesis along the catheter shaft up to the handle, wherein the handle comprises at least one spool to receive the proximal end of the preloaded wire with a proximal portion of the preloaded wire being coiled onto the spool.

## Description

### TECHNICAL FIELD

This invention relates to a system for delivery of an endoluminal prosthesis into a body lumen, especially into a blood vessel of a patient to restore patency of the vessel.

### BACKGROUND OF THE INVENTION

The present invention relates to a system for delivery of an endoluminal prosthesis into a body lumen, the system comprising a delivery catheter and a prosthesis to be delivered, the delivery catheter comprising a catheter tip, a catheter shaft, and a handle for deployment of the prosthesis, the prosthesis comprising a hollow-cylindrical main body and a wire extending through at least a portion of the main lumen of the prosthesis in a preloaded configuration.

Generally, endoluminal prostheses may include vascular prostheses, which are also referred to as vascular implants, and which are implanted for the treatment of aneurysms, occlusions, lesions or other damage in arteries. Such an endoluminal prosthesis can be a stent a stent graft or a combination thereof. The vascular prosthesis is introduced to the blood vessel by endoluminal techniques employing suitable delivery devices, in order to restore patency of the blood vessel across the lesion.

Systems comprising additional preloaded wires are usually employed in cases where once the prosthesis is delivered to the target site in a vessel a second access at a predetermined position of the prosthesis is required in a following step of the process of treatment of the damaged artery. Examples therefore are systems for treating lesions or occlusions of branched vessels or treatment of aneurysms in the thoracoabdominal artery, where an additional access to a second vessel or a side branch is required to ensure direction of blood flow through these side vessels, too. As it is often difficult and time consuming to access such side branches upon implantation of the prosthesis, an additional wire, a so-called preloaded wire, is preloaded with the prosthesis in such a manner that this preloaded wire ensures for example an exit through a fenestration in the prosthesis to a side branch of the artery.

EP2517671A1 for example discloses a system for deployment of an endoluminal prosthesis having a branch and a fenestration in the side wall of a main tubular graft body and a wire segment extending through the fenestration and through the lumen of the branch in a preloaded configuration. Such a system is used to treat aneurysms or lesions in the thoracoabdominal aorta, where a connection of the main aorta to the arteries branching off the aorta like e.g. the renal arteries has to be stablished to ensure blood flow. Placing these branches requires insertion of guide wires and delivery devices. This procedure may be very time-consuming and difficult to perform, as it is very complex to cannulate multiple fenestrations in the graft and the corresponding branch vessels when multiple smaller branch grafts are deployed. The preloaded wires allow quicker access to these side branches as time-consuming probing for the fenestrations can be avoided. However, a huge disadvantage of the systems disclosed in the art is the fact that depending on the procedure, very long preloaded wires are required to allow to pass the body and also allow for loading the respective guide catheters needed in the procedure. These long wires require additional personnel for the procedure to ensure its function and sterility. Further, using the system disclosed in EP2517671A1 either pivot fenestrations are required as for half of the side branches the bridging stents have to be inserted against the blood flow in the main prosthesis to a direction in blood flow in the side arm or an additional self-sealing fenestration in the side wall of the main body is required to allow the physician to enter all side branches in the direction of the blood flow. Both requirements add additional risk of failure of the procedure.

### SUMMARY OF THE DISCLOSURE

The object of the present invention is therefore to provide a system for delivery of an endoluminal prosthesis that allows effective, safe and less time-consuming implantation of an endoluminal prosthesis and the provision of a luminal access from the prosthesis to side branches or branched vessels. An effective and less time-consuming implantation of an endoluminal prosthesis is also associated with less exposure to irradiation of the patient, less use of contrast media and less traumatic interventions.

This object is achieved according to the present invention by a system for delivery of an endoluminal prosthesis into a body lumen of a patient as defined in claim 1.

The invention is therefore said to reside in a system for delivery of an endoluminal prosthesis, the system comprising a delivery catheter and a prosthesis to be delivered, the delivery catheter comprising a catheter tip, a catheter shaft, and a handle for deployment of the prosthesis, the prosthesis comprising a hollow-cylindrical main body having a side wall, a proximal opening and a distal opening and a main lumen extending therebetween, the system further comprising a wire extending through at least a portion of the main lumen of the prosthesis in a preloaded configuration, the wire extending in distal direction to a position in proximity to the distal opening of the prosthesis and the wire extending in proximal direction proximal of the proximal opening of the prosthesis along the catheter shaft up to the handle, wherein the handle comprises at least one spool to receive the proximal end of the preloaded wire with a proximal portion of the preloaded wire being coiled onto the spool.

Throughout the specification the term "proximal" generally denotes the position, direction or a portion or end of a component of the system that lies closer to the handle of the device or the physician treating the patient. Correspondingly the term "distal" means that position, direction or a portion or end of a component of the system according to the invention that is/leads further/furthest away from the handle of the device or the physician treating the patient.

### Prosthesis:

The endoluminal prosthesis to be delivered is a stent, a graft, a stent graft, a valve, or any combination thereof. In one embodiment, the endoluminal prosthesis is a vascular prosthesis also called a vascular implant.

The stent portion of the prosthesis can be made of any suitable material, such as metal, metal alloy, or polymeric material. Exemplary materials include stainless steel, nitinol, cobalt chromium alloy, ceramics and composites, bioabsorbable polymers, biostable polymers and thermotropic liquid crystal polymers. The stent can be fabricated by utilizing any number of methods known in the art. For example, a stent can be fabricated from a hollow or formed tube that is machined using lasers, electric discharge milling, chemical etching or other known techniques. Alternatively, the stent can be fabricated from a sheet that is rolled into a tubular member, or formed of a toroidal rings, wire or filament construction as known in the art. In a preferred embodiment of the present invention, the medical device comprises a metallic stent. Though the stent may also be made from any degradable or nondegradable polymer. Generally, the stent may be balloon-expandable or self-expandable. In a preferred embodiment, the stent is a self-expandable stent made of shape memory alloy like e.g. Nitinol.

In a further preferred embodiment, the prosthesis is a stent graft, the stent graft preferably being a metallic stent being covered with a graft material. Suitable graft materials include PTFE, expanded PTFE (ePTFE) or electrospun PTFE like for example Bioweb^{™} from Zeus Industrial Products, Inc.. In a further embodiment, the membrane may be made from polyester, polyurethane, polystyrene, polytetrafluoreth-ylene, ultra-high-molecular-weight polyethylene (UHMPE), or mixtures thereof. The membrane or cover or graft material of the stent graft may cover the whole stent or only a portion thereof. Further, the graft material may cover the stent on its outer side or its inner side, also called luminal side, or may cover the outside and the inside of the stent as a whole or only a portion thereof. There are many ways well known in the art to fix the graft material to the stent among them is sewing or gluing the membrane to the stent, spinning the graft material directly on the stent, or clamping the graft material between stent struts embodied to function as clamps.

In one embodiment, the endoluminal prosthesis comprises at least one fenestration in the side wall of the hollow-cylindrical main body. The term "fenestration" herein refers to an opening provided through the side wall of the prosthesis from the interior of the prosthesis to the exterior of the prosthesis. A fenestration may have a variety of geometries including circular, semi-circular, oval, oblong, or other geometries.

In another embodiment, the endoluminal prosthesis comprises at least one side branch, the side branch comprising a hollow-cylindrical side branch body having a side wall, a proximal opening and a distal opening and a side branch lumen extending therebetween, wherein the side branch lumen is in fluid communication with the main lumen.

In one embodiment, the at least one side branch of the endoluminal prosthesis extends from the at least one fenestration in the side wall of the hollow-cylindrical main body, the side branch having a first side branch opening and a second side branch opening and a side branch lumen extending between the first side branch opening and the second side branch opening, the first side-branch opening being firmly attached to the prosthesis material around the fenestration in the side wall.

In an alternative embodiment of the present invention, the at least one side branch of the endoluminal prosthesis is formed integral with the hollow-cylindrical main body of the prosthesis. The side wall of the hollow-cylindrical main body and the side wall of the side branch are made from one piece of material. In this case the opening in the side wall of the hollow cylindrical main body is not a fenestration cut into the wall of the hollow-cylindrical main body, but is an opening that results from the bifurcated shape of the prosthesis. An example for such a bifurcated prosthesis is described in EP 3920845 A1.

In one embodiment of the present invention, the side branch extends outside the main lumen from the fenestration or opening in the side wall of the hollow-cylindrical main body in direction of the blood flow.

In another embodiment of the present invention, the side branch extends into and within the main lumen of the prosthesis from the fenestration in the side wall of the hollow-cylindrical main body towards the blood flow. In an alternative embodiment, in case the hollow-cylindrical main body and the side wall of the side branch are made from one piece of material, the side branch extends into and within the main lumen of the prosthesis from the opening in the side wall of the hollow-cylindrical main body towards the blood flow. This means, the side branch is folded inwards into the main lumen of the main body of the prosthesis.

Such a design where the side branch extends into and within the main lumen of the prosthesis from the fenestration or opening in the side wall of the hollow-cylindrical main body towards the blood flow has the advantage that the opening of the branch lies within the transversal section of the main lumen toward the blood flow. Thus, a bridging stent can be easily placed within the side branch lumen of the prosthesis and reach out of the prosthesis into the side arms of an outgoing vessel. This concept also ensures the correct amount of blood dissecting in the side vessel by selecting the correct cross section of the side branch.

In a further embodiment, the endoluminal prosthesis comprises more than one, in particular two, three, four, five or six fenestrations in the side wall of the main lumen.

In further embodiments, the endoluminal prosthesis may be provided with side branches branching off the main body at a fenestration or opening either inside the main body lumen or outside the main body lumen. Any combination of unbranched fenestrations, branches branching off the fenestration or opening inside the main body lumen and branches branching off the fenestration or opening outside the main body lumen is included according to the present invention.

In one specific embodiment of the present invention, the endoluminal prosthesis is a prosthesis for treatment of thoracoabdominal aneurysms having four fenestrations or four side branches or a combination of fenestrations and side branches to sum up to four.

In another specific embodiment of the present invention, the endoluminal prosthesis is a prosthesis for treatment of thoracoabdominal aneurysms having five fenestrations or five side branches or a combination of fenestrations and side branches to sum up to five.

In an even more specific embodiment of the present invention, the endoluminal prosthesis is a preferably self-expanding vascular prosthesis or stent graft comprising a hollow-cylindrical main body with a longitudinal axis, a proximal opening and a distal opening, and also a main lumen extending between the proximal opening and the distal opening, the main body having a proximal end portion and a distal end portion, which respectively comprise the first opening and the second opening, and also a middle portion arranged between the proximal end portion and the distal end portion; and also comprising at least four side branches, which extend, towards the distal direction, into and within the main lumen from fenestrations or openings in the prosthesis material, each side branch having a first side-branch opening and a second side-branch opening and also a side-branch lumen extending between the first side-branch opening and the second side-branch opening, wherein the side branch lumen is in fluid communication with the man lumen.

In a further embodiment, the side branches are arranged in a middle portion of the endoluminal prosthesis where the middle portion has a smaller or equal diameter in comparison to the diameter of the end portions of the prosthesis.

In another embodiment of the present invention, the endoluminal prosthesis is a prosthesis for treatment of a lesion in the aortic arch, the endoluminal prosthesis having one, two or three fenestrations or side branches or a combination of fenestrations and side branches to sum up to three.

In another further embodiment of the present invention, the endoluminal prosthesis is a prosthesis for treatment of a lesion in the iliac artery, the endoluminal prosthesis having one fenestration or side branch.

In a preferred embodiment, the prosthesis is preferably a stent graft, comprising stent rings made of a self-expanding material, preferably a shape-memory material as e.g. Nitinol and a membrane attached thereto which preferably comprises or is made of a material selected from polyester, polyurethane, polystyrene, polytetrafluoroethylene, ultra-high-molecular-weight polyethylene (UHMPE), or mixtures thereof. A more detailed description of such a prosthesis can be found for example in US 10952840 B2, the entire disclosure of which, except for any definitions, disclaimers, disavowals, and inconsistencies, is incorporated herein by reference.

### Preloaded wires:

The preloaded wire can be arranged along the prosthesis in any manner suitable for the application intended. In one embodiment, the preloaded wire extends from the spool at the handle at the proximal end of the catheter through the proximal opening of the main body and through the distal opening of the main body to a position in proximity to the distal opening of the prosthesis.

In a first preferred embodiment, the preloaded wire extends from the spool at the handle at the proximal end of the catheter to a position outside the main lumen of the prosthesis in a proximal region of the prosthesis, further extends through a fenestration in the side wall of the hollow-cylindrical main body into the lumen of the prosthesis, and extends further inside the lumen of the main body to a position in proximity to the distal opening of the prosthesis.

In a second preferred embodiment, the preloaded wire extends from the spool at the handle at the proximal end of the catheter to a position outside the main lumen of the prosthesis in a proximal region of the prosthesis, further extends through the fenestration in the side wall of the hollow-cylindrical main body and through the side branch lumen into the main lumen of the prosthesis, and extends further inside the lumen of the main body to a position adjacent to or distal of the distal end of the prosthesis.

It has to be noted that in case the endoluminal prosthesis has more than one fenestration or side branch, each fenestration or side branch may be or may not be provided with a preloaded wire.

In a further preferred embodiment of the present invention, the endoluminal prosthesis has at least two fenestrations. At least one preloaded wire extends from the spool at the handle at the proximal end of the catheter to a position outside the main lumen of the prosthesis in a proximal region of the prosthesis, further extends through a first fenestration in the side wall of the hollow-cylindrical main body, extends further inside the lumen of the main body to a second fenestration in the side wall of the hollow-cylindrical main body extends through the second fenestration, and extends further outside the lumen of the main body to a position proximal of the proximal end of the prosthesis adjacent to the handle, thus forming a loop.

It is readily understood by the person skilled in the art that in a further embodiment of the endoluminal prosthesis, one or more of the fenestrations may be provided with a side branch either inside or outside of the main lumen of the prosthesis or the prosthesis may be provided with side branches formed integrally with the main body. In these embodiments, the preloaded wire passes the lumen of the side branch.

In one of these further embodiments, where the side branches are located inside the main lumen of the prosthesis, the preloaded wire extends from the spool at the handle at the proximal end of the catheter to a position outside the main lumen of the prosthesis in a proximal region of the prosthesis, further extends through a first fenestration or opening in the side wall of the hollow-cylindrical main body and through a first side branch lumen into the main lumen of the prosthesis, and extends further inside the lumen of the main body to a position to a second side branch opening, passes along the lumen of the second side branch to a second fenestration or opening in the side wall of the hollow-cylindrical main body, extends through the second fenestration or opening, and extends further outside the lumen of the main body to a position proximal of the proximal end of the prosthesis adjacent to the handle, thus forming a loop.

In another embodiment of the present invention, the prosthesis comprises at least one fenestration or side branch and an ancillary fenestration in the side wall of the hollow-cylindrical main body. The ancillary fenestration is a small fenestration not meant to allow access to a side artery but to allow a preloaded wire to extend from the spool at the handle at the proximal end of the catheter to a position inside the main lumen of the prosthesis in a proximal region of the prosthesis, the preloaded wire further extending through the ancillary fenestration in the side wall of the hollow-cylindrical main body of the main lumen of the prosthesis, extending outside of the lumen of the main body to the fenestration or side branch in the side wall of the hollow-cylindrical main body, the preloaded wire further extending through the fenestration or side branch into the main lumen of the hollow-cylindrical main body and extending further to a position adjacent to or distal of the distal end of the prosthesis.

In a preferred embodiment of the present invention, the prosthesis comprises two, three, four, five or more side branches which extend from two, three, four, five or more fenestrations or openings in the side wall of the hollow-cylindrical main body, respectively, each side branch receives a preloaded wire. Preferably, each preloaded wire extends from the spool at the proximal end of the catheter to a position outside the main lumen of the prosthesis in a proximal region of the prosthesis, further extends into the side branch lumen and further into the main lumen of the prosthesis, and extends further inside the lumen of the main body to a position distal of the distal end of the prosthesis.

The preloaded wire is a highly elastically, flexible, resilient wire which is not plastically deformable, biocompatible, and non-breakable. The wire can be narrowly coiled around the spool but still returns to a straight configuration once uncoiled from the spool. Suitable wires may be made from any highly elastic material, but are preferably made from material including Nitinol. Suitable wires to be used as preloaded wire also include guide wires available in the market. The preloaded wire may be a solid wire, a wire having a solid core wire where the core wire is encased by one or more metal spring coils, a ribbon wire where the spring coil encases both, a core wire and a ribbon wire or any combination thereof.

To ensure non-harming of the vessel to be treated by the preloaded wire, the distal tip of the wire has to be atraumatic and flexible. In a preferred embodiment, the distal tip of the preloaded wire is grinded to form a cone-shaped tip with a rounded distal end.

In a preferred embodiment, at least the distal tip of the preloaded wire is visible under x-ray. In one embodiment, the tip of a nitinol wire is provided with a wire made from radiopaque material such as Tungsten which is wrapped around the tip of the preloaded wire. In a preferred embodiment, the preloaded wire is a solid nitinol wire being provided with a distal tip that is grinded down and encased with a tungsten coil.

In various occasions, depending on the medical procedure to be performed, the distal tip of the preloaded wire has to be caught or snared by a lasso or snare device to be able to use the wire as a retrograde wire. Accordingly, the distal tip of the preloaded wire is configured to be easily captured but not deformed while captured.

### Catheter features:

As depicted above the system for delivering an endoluminal prosthesis according to the present invention comprises a delivery catheter which comprises a catheter tip, a catheter shaft, and a handle for deployment of the prosthesis. The prosthesis is mounted to the catheter shaft on a portion just proximal of the distal end of the catheter. The distal end of the preloaded wire extends distally to a position in proximity to the distal opening of the prosthesis.

In a preferred embodiment of the present invention, the preloaded wire is releasably captured within the catheter tip at the distal end of the catheter. In an alternative preferred embodiment, the distal end of the preloaded wire ends within the main lumen of the prosthesis at a position adjacent to the distal end of the prosthesis and is captured underneath the prosthesis in its delivery configuration.

In operation, once the prosthesis is expanded, the distal end of the preloaded wire is floating loosely either within the lumen of the main body of the prosthesis or within the lumen of the side branch or within the body lumen distal of the distal end of the prosthesis and can be captured by a lasso or a snare device introduced into the lumen from either the distal site of the prosthesis or the proximal site. The preloaded wire will then be uncoiled and its distal end will be torn out of the body distally in order the preloaded wire to serve as a guide for introduction of a guide catheter through the fenestration or side branch of the prosthesis to allow bridging of the side branch of the prosthesis and the side branch of the arterial side arm.

The delivery catheter further may comprise a guidewire lumen to receive a guidewire. The guide wire lumen extends from the proximal end to the distal end of the catheter in an "over the wire" mode or alternatively the guide wire lumen extends from a position proximal of the prosthesis mounted on the catheter but distal of the proximal end of the catheter to the distal end of the catheter in an "rapid exchange" mode which are both well known in the art.

At the proximal end of the catheter a handle is mounted which comprises the spool for the preloaded wire.

### Handle/Spool features:

The handle comprises a spool to receive the proximal end of the preloaded wire. A proximal portion of the preloaded wire is coiled onto the spool. The preloaded wire runs from the spool at the proximal end of the catheter through the handle along the catheter shaft to the prosthesis. The handle is provided with a suitable seal to seal off the entrance of the preloaded wires to the catheter system.

In order to avoid blood leaking out of the system and the vessel during placement of the endoluminal prosthesis the handle comprises at least one seal liquid tightly sealing the exit of the preloaded wire out of the system towards the spool. In a preferred embodiment of the present invention, the seal is a sealing ring or sealing disk sealing the wire. In an alternative embodiment of the present invention, the seal is a lip seal or a duckbill seal. In an even more preferred embodiment, the handle comprises two seals in a series. The first seal is a ring seal as depicted above, sealing the exit of the preloaded wire out of the handle and/or system. The second seal is a lip seal or a duckbill seal. This second seal lying inside of the handle allows sealing of the system once the preloaded wire is fully retracted from the system. The combination of a ring seal and a lip seal ensure liquid tight closure of the system during all steps of the implantation procedure of the endoluminal prosthesis. Such a double seal is provided for each exit of a preloaded wire from the system or handle.

In a preferred embodiment, the proximal end of the preloaded wire is releasably fixed to the spool via fitting the wire into a recess in the spool. Though it is readily understood by the person skilled in the art that the proximal end of the wire may be affixed to the spool in any suitable manner, like gluing, welding or friction fitting.

The spool itself is coupled to the handle. In one embodiment, the spool is attached to the handle via a retainer. In a preferred embodiment, the spool is releasably attached to the handle. In a further preferred embodiment, the spool is attached to the handle by a releasable connector. In an alternative embodiment, the spool is attached to the handle via a retainer which itself is attached to the handle by a releasable connector.

By releasably fixing the spool and/or the retainer to the handle, the spool can be detached from the handle. Such a detachable fixation can be embodied in any suitable manner known in the art like for example and not limitation a bayonet lock, a click closure, or a locking button.

This is advantageous for better handling of the delivery device. The spool containing the preloaded wire can thus be detached once the preloaded wire is retracted from the system or is not required in the procedure any more. This way, the system as a whole will be less bulky and easier to handle. Also, the spool can be detached to allow working on the spool and the handle by two physicians at the same time.

The spool itself can be formed in any suitable manner to wind up the preloaded wire. In a preferred embodiment, the spool comprises a detachable lid to ensure the proper winding up of the preloaded wire. Winding the preloaded wire upon the spool allows easy handling of the wire. Once released from the distal tip of the catheter the distal end of the preloaded wire can be uncoiled from the spool and be pulled further distally of the distal end of the catheter to be captured by a snare device and to allow for guiding a guide catheter from the distal end portion of the preloaded wire to a position adjacent to a desired treatment site in the endoluminal prosthesis. On the other hand, the preloaded wire can be coiled on the spool and can thus be retracted from the desired treatment site or can be fully coiled on the spool and thus fully retracted from the vessel.

In a system comprising a prosthesis comprising two, three, four, five, or more fenestrations or side branches, each fenestration or side branch may be provided with its own preloaded wire. Each preloaded wire runs from a position in proximity to the distal opening of the prosthesis to a spool attached to the handle at the proximal end of the catheter. In one embodiment, each spool is fixed to the handle separately via a fixed retainer or alternatively via a retainer and a releasable spool connector.

In another embodiment, two, three, four, five, or more spools are arranged in parallel and are attached to the handle via one fixed retainer or alternatively via one retainer and releasable spool connector for all spools. If more than one spool is employed in parallel the spools are moveable independently of each other. In a preferred embodiment of the present invention, neighboring spools work in an opposite winding direction to each other to make sure the preloaded wires are placed at the handle with sufficient distance to each other to enable easy and convenient operation of each spool separately.

In a preferred embodiment, the endoluminal prosthesis is a prosthesis for repair of a thoracoabdominal aortic aneurysms and comprises four side branches which extend from four fenestrations in the side wall of the hollow-cylindrical main body into the lumen of the main body. Each side branch receives a preloaded wire. The handle of the delivery device comprises four spools, each spool receiving one preloaded wire. Each preloaded wire runs from the spool where a proximal portion of the preloaded wire is coiled on through the handle along the catheter shaft outside of the main lumen of the endoluminal prosthesis to one fenestration or opening in the side wall of the main body of the prosthesis, enters the fenestration, runs inside the lumen of the side branch to a position in proximity to the distal opening of the main lumen of the prosthesis.

In an even more preferred embodiment, two times two spools at a time are coupled to the handle via one retainer, while each of the two times two spools can be operated separately. In an even further preferred embodiment, two spools coupled to the handle via one retainer work in opposite winding directions.

### BRIEF DESCRIPTION OF THE DRAWINGS

Exemplary embodiments of the invention are represented in the drawings and are described in more detail below with reference thereto.
FIG. 1 shows a system according to the present invention;
FIG. 2 shows an endoluminal prosthesis provided with preloaded wires according to the invention;
FIG. 3 shows a schematic view of a portion of the handle of the delivery device according to the present invention including a spool for the preloaded wire to be coiled onto;
FIG. 4 shows a schematic view of a portion of the handle of the delivery device according to another embodiment of the present invention including a spool for the preloaded wire to be coiled onto.

### DESCRIPTION OF PREFERRED EMBODIMENTS

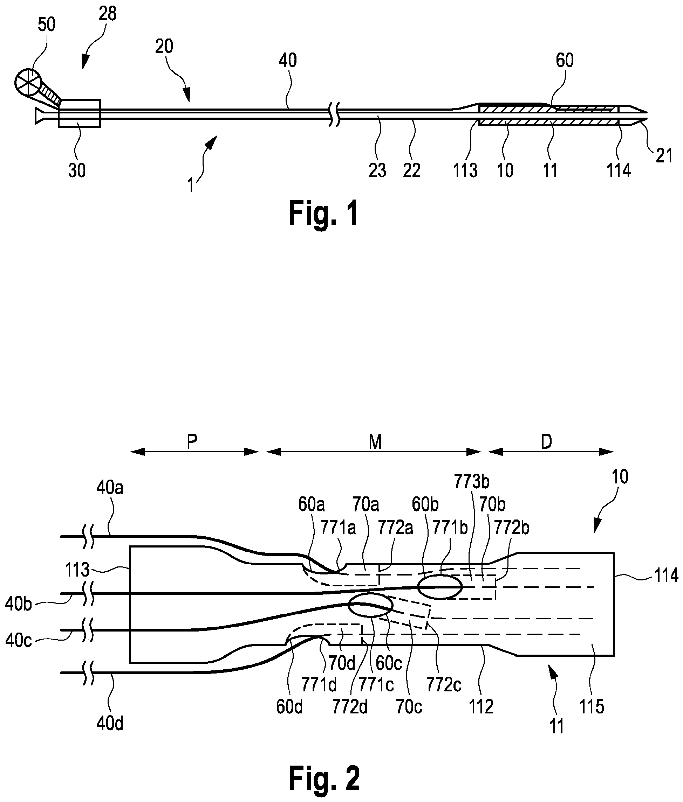

FIG. 1 depicts an exemplary embodiment of a system (1) of the present invention. The system (1) for delivery of an endoluminal prosthesis comprises a delivery catheter (20) and a prosthesis (10) to be delivered. The delivery catheter (20) comprises a catheter tip (21), a catheter shaft (22), and a handle (30) for deployment of the prosthesis. Optionally, the catheter further comprises a guidewire lumen (23) to receive a guidewire (not shown). The prosthesis which is mounted to the catheter in a distal region of the catheter shaft (22) and proximal to the catheter tip (21) comprises a hollow-cylindrical main body (11) having a side wall (112), a proximal opening (113) and a distal opening (114) and a main lumen (115) extending therebetween. The system (1) further comprises a wire (40) extending through at least a portion of the main lumen (115) of the prosthesis (10) in a preloaded configuration, the wire extending distally to a position in proximity to the distal opening (114) of the prosthesis (10) and the wire extending proximal of the proximal opening (113) of the prosthesis along the catheter shaft (22) up to a handle (30). The handle (30) comprises at least one spool (50) to receive the proximal end of the preloaded wire (441). A proximal portion of the preloaded wire (40) is coiled onto the spool (50).

While in Figure 1 the distal end of the preloaded wire (40) is located adjacent to the distal opening (114) of the prosthesis (10), the distal of the preloaded wire (40) may be releasably captured within the catheter tip (21) in an alternative embodiment.

As depicted above, the prosthesis itself may be a bare stent, a graft or a stent graft or any combination thereof. The prosthesis (10) depicted in Figure 1 comprises one fenestration (60) in the side wall (112) of the hollow-cylindrical main body (11). As depicted in Fig. 1 the preloaded wire (40) extends from the spool (50) at the proximal end of the catheter (28) to a position outside the main lumen (115) of the prosthesis in a proximal region of the prosthesis, extending through the fenestration (60) in the side wall (112) of the hollow-cylindrical main body (11) into the lumen of the prosthesis (115), and extending further inside the lumen of the main body (115) to a position adjacent to the distal end of the prosthesis (114).

Generally, the prosthesis does not need to comprise a fenestration. Though, it is preferred that the prosthesis comprises at least one fenestration. Alternatively or additionally, the prosthesis may comprise a side branch (70) that extends from a fenestration (60) in the side wall (112) of the hollow-cylindrical main body (11), the side branch (70) having a first side branch opening (771)and a second side branch opening (772) and a side branch lumen (773) extending between the first side branch opening (771) and the second side branch opening (772), the first side-branch opening (771) being firmly attached to the prosthesis material around the fenestration (60) in the side wall (112) of the of hollow-cylindrical main body (11). Such a side branch (70) may extend distally into and within the main lumen (115) from the fenestration (60) in the side wall of the hollow-cylindrical main body (11). In the latter case the preloaded wire (40) extends from the spool (50) at the proximal end of the catheter (28) to a position outside the main lumen (115) of the prosthesis (10) in a proximal region of the prosthesis, extending through the fenestration (60) in the side wall of the hollow-cylindrical main body (11) and through the side branch lumen (773) into the main lumen (115) of the prosthesis, and extending further inside the lumen (115) of the main body (11) to a position distal of the distal end of the prosthesis (114).

In an alternative embodiment, the prosthesis comprises four side branches (70a, b, c, d) which extend from four fenestrations (60a, b, c, d) in the side wall (112) of the hollow-cylindrical main body (11), each side branch receiving a preloaded wire (40a, b, c, d).

An embodiment of an endoluminal prosthesis (10) employed in the present invention is shown schematically in Figure 2. It has to be understood that the prosthesis in general has a compressed delivery configuration when mounted on the catheter and an expanded configuration upon implantation in a body conduct. The prosthesis depicted in Figure 2 is shown in its expanded configuration.

This prosthesis is designed for the repair of a thoracoabdominal aortic aneurysm. Its main body (11) has three portions, a proximal portion (P), a distal portion (D), and a middle portion (M) therebetween, wherein the middle (M)portion has a reduced diameter compared to the distal (D) and the proximal portion (P). The prosthesis (10) comprises four side branches (70 a, b, c, d) which extend from four fenestrations (60 a, b, c, d) in the middle portion (M) of the side wall (112) of the hollow-cylindrical main body (11) into the lumen (115) of the main body. Each side branch (70 a, b, c, d) receives a preloaded wire (40 a, b, c, d). The handle (30) of the delivery device (not shown) comprises four spools (50 a, b, c, d) (not shown), each spool receiving one preloaded wire (40). Each preloaded wire (40 a, b, c, d) runs from the spool (50) where a proximal portion of the preloaded wire (40) is coiled on through the handle (30) along the catheter shaft (22) outside of the main lumen (115) of the endoluminal prosthesis (10) to one fenestration (60 a, b, c, d) in the side wall (112) of the hollow-cylindrical main body of the prosthesis, enters the fenestration (60 a, b, c, d), runs inside the lumen of the side branch (773 a, b, c, d) to a position inside the main lumen (115) adjacent to the distal opening (114) of the main lumen (115) of the prosthesis (10).

There are several advantages of the system for repair of a thoracoabdominal aortic aneurysm according to the present invention over the art. First, the prosthesis, which is preferably self-expanding provides a vascular prosthesis system with which thoracoabdominal aneurysms can be successfully bridged, while at the same time reliably ensuring the supply of blood to the branching-off side vessels. This is achieved by connecting further connection stents/stent grafts, being routed in the interior of the main lumen of the vascular prosthesis. It is ensured by the inwardly routed branch-off of the side branches/arms that there is sufficient space for the vascular prosthesis in the vessel in which the vascular prosthesis is released, but at the same time the function of the arms/side branches is ensured. The branches branching off inwardly have the advantage of a longer overlapping zone with connection stents/stent grafts for the branching-off side vessels. Also, as a result of the specific arrangement of the side branches branching off inwardly into the main lumen, the system is quite flexible and consequently the prosthesis does not have to be individually made up in each case for the patient specifically to be treated, but instead can be prefabricated, particularly for a large number of patients, on account of its specific arrangement and dimensions of the side branches.

Further, the preloaded wires allow for relatively easy and quick access to the side arms or branching-off side vessels. With each side branch comprising its own preloaded wire, quicker access to the side branches is allowed, as time-consuming probing for the fenestrations can be avoided. Also, access to the side arms is in the direction of the blood flow for each side branch, allowing to place the bridging stents in a smooth manner and reducing risk of failure when re-connecting to side arms of the vessel to the blood flow.

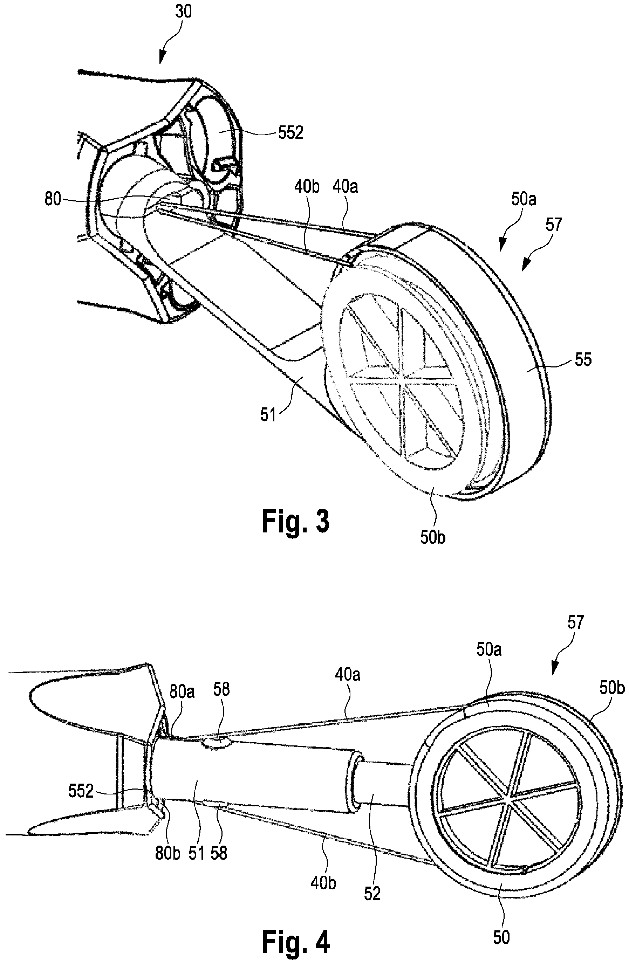

Figures 3 and 4 show drawings of two exemplary embodiments of a portion of the handle (30) and a spool arrangement (57), which is attached to the handle (30) via a retainer (51). The spool arrangement (57) may be either one single spool (50) or may contain two spools arranged in parallel (50a, 50b) as depicted in Figure 3. The spool (50) and/or the retainer (51) is fixed to the handle. In Fig. 3 the spool (50) and/or retainer (51) is fixedly attached to the handle (30) at a gadget (552) in the handle to receive the retainer (51) of the spool. The retainer can be fixed to the handle by any known suitable means, e.g. By gluing or by any mechanical connection means like e.g. a snap closure or a screw threat. The handle (30) shown in Figure 3 is designed to receive two times two spools arranged in parallel. Figure 3 shows two spools (50a, 50b) coupled to the handle (30) via one retainer (51). The handle (30) shown is further equipped with a second gadget (552) to receive a third spool or a set of two spools at a time. As depicted in Figure 3 two spools (50a, 50b) at a time are coupled to the handle (30) via one retainer (51), while each spool (50a, 50b) can be operated separately. While the handle (30) is shown to be capable of receiving four spools, it is obvious to the person skilled in the art, that the handle (30) can readily be designed to hold one single spool, two single spools or one double spool (i.e. a set of two spools) and a single spool or two double spools.

In an alternative embodiment (not shown), each retainer may hold two, three, four, five, or more spools in parallel, where each spool can be operated independently of the other spools.

In an even more preferred embodiment of the present invention, the spools held via one retainer are arranged in parallel but in opposite working direction. That means, the first spool winds up the first preloaded wire in a first winding direction, while the second neighboring spool winds up the second preloaded wire in a second winding direction opposite to the first winding direction. These opposite winding direction results in opposite positions of the preloaded wire, thus allowing easy and convenient handling of the different preloaded wires.

Each spool (50) receives one preloaded wire (40). The proximal end of the preloaded wire (441) may be releasably fixed to the spool via fitting the wire into a recess in the spool (not shown). A proximal portion of the preloaded wire (40) is coiled onto the spool (50). As can be seen, the preloaded wires (40a, 40b) enter the handle (30), which itself is provided with a seal (80), and extend further along the catheter to the prosthesis (not shown).

The spool (50) is covered by a detachable lid (55) to ensure the preloaded wire (40) to stay coiled on the spool (50). In operation, the preloaded wire (40) can be coiled onto the spool (50) or can be uncoiled from the spool (50), depending on the need where the distal end of the preloaded wire (40) is needed. With the preloaded wire (40) being coiled onto the spool (50), the whole system is very compact and easy to handle. No long wires have to be handled and kept sterile during the implantation procedure. Also, the spools can be detached from the handle (30) allowing separate operation of catheter and preloaded wire (40). Furthermore, if not required anymore, the preloaded wire (40) can be withdrawn (coiled up) completely and the spool carrying the wire can be removed from the handle.

Figure 4 shows a drawing of a portion of the handle (30) and a spool arrangement (57), which is attached to the handle (30) via a retainer (51) similar to the embodiment shown in Fig 3. However, the spool arrangement (57) depicted in Figure 4 is attached to the handle via a retainer and a releasable spool connector (52). As is true for the embodiment according to Figure 3, the spool arrangement (57) may be either one single spool (50) or may contain two spools arranged in parallel (50a, 50b) as depicted in Figure 3. As disclosed above, the spool arrangement may also contain tree, four, five or more spools in parallel.

As can be seen, the winding direction of the two spools (50a, 50b) shown in Figure 4 are opposite to each other resulting in the preloaded guidewires (40a, 40b) to run along on opposite sides of the retainer, thus facilitating handling, winding up und unwinding the single preloaded wire during the implantation process of the endo-vascular prosthesis.

In the embodiment depicted in Figure 4, the releasable spool connector (52) is configured in a way that the spool assembly (57) may be detached from the handle via a click closure including spring buttons (58). In alternative embodiments, the releasable connector is embodied in any suitable manner known in the art like for example and not limitation a bayonet lock or a click closure.

While the disclosed subject matter is described herein in terms of certain embodiments, those skilled in the art will recognize that various modifications and improvements can be made to the disclosed subject matter without departing from the scope thereof. Moreover, although individual features of one embodiment of the disclosed subject matter can be discussed herein or shown in the drawings of the one embodiment and not in other embodiments, it should be apparent that individual features of one embodiment can be combined with one or more features of another embodiment or features from a plurality of embodiments.

In addition to the various embodiments depicted and claimed, the disclosed subject matter is also directed to other embodiments having any other possible combination of the dependent features claimed below and those disclosed above. As such, the particular features presented in the dependent claims and disclosed above can be combined with each other in other manners within the scope of the disclosed subject matter such that the disclosed subject matter should be recognized as also specifically directed to other embodiments having any other possible combinations. Thus, the foregoing description of specific embodiments of the disclosed subject matter has been presented for purposes of illustration and description. It is not intended to be exhaustive or to limit the disclosed subject matter to those embodiments disclosed.

Many modifications, variations, or other equivalents to the specific embodiments described above will be apparent to those familiar with the art. It is intended that the scope of this disclosed subject matter be defined by the claims below and those modifications, variations and equivalents apparent to practitioners familiar with this art. Thus, it is intended that the disclosed subject matter include modifications and variations that are within the scope of the appended claims and their equivalents.

List of reference signs:
system (1)
endoluminal prosthesis (10)
delivery catheter (20)
catheter tip (21)
catheter shaft (22)
proximal end of the catheter (28)
handle (30)
hollow-cylindrical main body (11)
side wall of hollow-cylindrical main body (112)
proximal opening (113)
distal opening (114)
main lumen (115)
preloaded wire (40)
spool (50)
spool arrangement (57)
proximal end of the preloaded wire (40) (441)
fenestration (60)
side branch (70)
first side branch opening (771)
second side branch opening (772)
side branch lumen (773)
guidewire lumen (23)
spool retainer (51)
releasable spool connector (52)
detachable lid (55)
spring button (58)
gadget to receive a spool (552)
seal (80)

## Claims

1. A system for delivery of an endoluminal prosthesis, the system comprising:
a delivery catheter and a prosthesis to be delivered,
the delivery catheter comprising a catheter tip, a catheter shaft, and a handle for deployment of the prosthesis,
the prosthesis comprising a hollow-cylindrical main body having a side wall, a proximal opening and a distal opening and a main lumen extending therebetween,
the system further comprising a wire extending through at least a portion of the main lumen of the prosthesis in a preloaded configuration, the wire extending in distal direction to a position in proximity to the distal opening of the prosthesis and the wire extending in proximal direction proximal of the proximal opening of the prosthesis along the catheter shaft up to the handle,
wherein the handle comprises at least one spool to receive the proximal end of the preloaded wire with a proximal portion of the preloaded wire (40) being coiled onto the spool.

2. The system according to claim 1, wherein the prosthesis is a stent, a graft, a stent graft or any combination thereof.

3. The system according to claim 1 or 2, wherein the prosthesis comprises at least one fenestration in the side wall of the hollow-cylindrical main body.

4. The system according to any one of claims 1 to 3, wherein the prosthesis comprises at least one side branch, the side branch comprising a hollow-cylindrical side branch body having a side wall, a proximal opening and a distal opening and a side branch lumen extending therebetween, wherein the side branch lumen is in fluid communication with the main lumen.

5. The system according to claim 3, wherein the preloaded wire extends from the spool at the proximal end of the catheter to a position outside the main lumen of the prosthesis in a proximal region of the prosthesis, extending through the fenestration in the side wall of the hollow-cylindrical main body into the lumen of the prosthesis, and extending further inside the lumen of the main body to a position in proximity to the distal end of the prosthesis.

6. The system according to claim 4, wherein the preloaded wire extends from the spool at the proximal end of the catheter to a position outside the main lumen of the prosthesis in a proximal region of the prosthesis and further extending into the side branch lumen.

7. The system according to claim 6, wherein the preloaded wire extends from the spool at the proximal end of the catheter to a position outside the main lumen of the prosthesis in a proximal region of the prosthesis, extending into the side branch lumen, and extending further inside the side branch lumen into the main lumen of the prosthesis to a position in proximity to the distal end of the prosthesis.

8. The system according to any one of the preceding claims, wherein the prosthesis comprises four side branches, each side branch having a side branch lumen, each side branch lumen being in fluid communication with the main lumen and each side branch lumen receiving a preloaded wire.

9. The system according to any one of the preceding claims, wherein the delivery catheter further comprises a guidewire lumen to receive a guidewire.

10. The system according to any one of the preceding claims, wherein the spool is attached to the handle via a retainer.

11. The system according to any one of the preceding claims, wherein the spool and/or the retainer is detachable from the handle.

12. The system according to claim 11, wherein the spool is attached to the retainer by a releasable connector.

13. The system according to any of the preceding claims, wherein the endoluminal prosthesis comprises four side branches, each side branch receiving a preloaded wire and wherein the handle comprises four spools, each spool receiving one preloaded wire.

14. The system according to claim 13, wherein two spools at a time are coupled to the handle via one retainer, while each spool can be operated separately.

15. The system according to any one of the preceding claims, wherein the handle comprises at least one seal per preloaded wire accommodated in the system.
